# EUROPÄISCHE PATENTSCHRIFT

(11) **EP 1 467 704 B1**
(45) Veröffentlichungstag und Bekanntmachung des Hinweises auf die Patenterteilung: **24.01.2007**
(21) Anmeldenummer: 02795248.0
(22) Anmeldetag: 20.12.2002
(51) Int. Cl.: A61K 8/46, A61K 8/49, A61K 8/60, A61Q 5/02, A61Q 19/10

(54) **HAARKOSMETISCHE REINIGUNGSMITTEL MIT GUTEN SCHAUM- UND PFLEGEEIGENSCHAFTEN**
COSMETIC CLEANING AGENT FOR THE HAIR WITH EXCELLENT FOAMING AND HAIRCARE PROPERTIES
PRODUIT NETTOYANT COSMETIQUE POUR LES CHEVEUX PRESENTANT UN BON POUVOIR MOUSSANT ET OFFRANT UNE BONNE CAPACITE DE SOIN

(30) Priorität: 09.01.2002 DE 10200474
(43) Veröffentlichungstag der Anmeldung: 20.10.2004
(73) Patentinhaber: Beiersdorf AG, 20245 Hamburg (DE)
(72) Erfinder: ARGEMBEAUX, Horst, 21465 Wentorf (DE); HEITMANN, Birgit, 22769 Hamburg (DE); PRIMMEL, Bettina, 20146 Hamburg (DE); KAISER, Elke, 22769 Hamburg (DE)
(86) Internationale Anmeldenummer: PCT/EP2002/014613
(87) Internationale Veröffentlichungsnummer: WO 2003/057185

(56) Entgegenhaltungen:
- EP-A- 0 965 322
- EP-A- 1 043 010
- DE-A- 19 516 108
- US-A- 5 773 595

## Beschreibung

Die Erfindung betrifft kosmetische oder dermatologische Reinigungs- und Pflegezubereitungen, insbesondere zur Haarreinigung und -pflege. Die Zubereitungen enthalten eine synergistische Kombination aus anionischen und nichtionischen Tensiden. Die Zubereitungen sind mild zu Haut und Haaren und weisen gute Schaum- und Haarpflegeeigenschaften auf.

Zubereitungen dieser Art sind beispielsweise als Schaum- und Duschbäder, feste und flüssige Seifen oder sogenannte "Syndets" (synthetische Detergentien), Shampoos, Handwaschpasten, Intimwaschmittel, spezielle Reinigungsmittel für Kleinkinder und dergleichen bekannt.

Oberflächenaktive Stoffe - am bekanntesten die Alkalisalze der höheren Fettsäuren, also die klassischen "Seifen" - sind amphiphile Stoffe, die organische unpolare Substanzen in Wasser emulgieren können.

Diese Stoffe schwemmen nicht nur Schmutz von Haut und Haaren, sie reizen, je nach Wahl des Tensids oder des Tensidgemisches, Haut und Schleimhäute mehr oder minder stark.
Das gebräuchlichste Tensid für kosmetische Zusammensetzungen ist das Natriumlaurylethersulfat. Obwohl es gute Waschkraft besitzt und gut haut- und schleimhautverträglich ist, sollten empfindliche Personen den häufigen Kontakt damit meiden.

Es ist zwar eine große Zahl recht milder Tenside erhältlich, jedoch sind die Tenside des Standes der Technik entweder mild, reinigen aber schlecht, oder aber sie reinigen gut, reizen jedoch Haut oder Schleimhäute.

Diesem Übelstande gilt es also, Abhilfe zu schaffen.

Die vorliegende Erfindung betrifft insbesondere waschaktive haarkosmetische Zubereitungen, landläufig als Shampoos bezeichnet. Insbesondere betrifft die vorliegende Erfindung haarkosmetische Wirkstoffkombinationen und Zubereitungen zur Pflege des Haars und der Kopfhaut.

Auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche Haarbestandteile (z.B. Harnstoff, Harnsäure, Xanthin, Keratin, Glycogen, Citronensäure, Milchsäure) durch die Haarwäsche herausgelaugt werden.

Der Stande der Technik lässt es aber an Shampooformulierungen mangeln, welche dem geschädigten Haar in befriedigender Weise Pflege zukommen ließen. Aufgabe ist es daher, auch diesen Nachteilen des Standes der Technik Abhilfe zu schaffen.

Reinigungsmittel für Haut und Haare müssen eine gute Verträglichkeit aufweisen. Wird man diesem Anspruch gerecht mangelt es aber häufig an einer guten Schaumcharakteristik, schlechtem Schaumvolumen und/oder Cremigkeit des Reinigungsmittels. Gerade diese Eigenschaften werden vom Verbraucher als angenehm empfunden und er verbindet mit diesen subjektiven Eindrücken ein gutes Reinigungs- und Pflegepotential des kosmetischen Mittels.

Nächstliegender Stand der Technik bildet EP 1043010. Hierin werden milde Reinigungszubereitungen mit guten Schaumeigenschaften beschrieben. Diese Eigenschaften werden durch eine Tensidkombination von besonders amphoteren Tensiden, Alkylethem, Sulfosuccinaten und PEG-Sorbitanen erhalten. Zubereitungen ohne amphotere Tenside werden nicht offenbart. Das Schaumverhalten, die Verdickbarkeit und auch die Haut- und besonders die Augenschleimhautverträglichkeit von Zubereitungen, die amphoteren Tenside enthalten, ist vielfach nur mäßig. Aus diesem Grund werden amphotere Tenside fast ausschließlich in Verbindung mit anderen Tensiden eingesetzt, wodurch diese Eigenschaften teilweise synergistisch verbessert werden sollen ("Kosmetik", W. Umbach, Thieme Verlag 1988, Stuttgart).
Die aus EP 1043010 bekannten Reinigungszubereitungen sind bezüglich Verträglichkeit und Schaumeigenschaft daher verbesserungswürdig

US-5 773 595 offenbart kosmetische Reinigungszusammensetzungen, die Alkyl glycoside, Alkyl(ether) sulfate und Tenside vom Siccinat-Typ enthalten.

Die Aufgabe der vorliegenden Erfindung ist daher auch eine alternative kosmetische Reinigungs- und Pflegezubereitung bereit zu stellen, die sich insbesondere durch gute Haut- und Haarverträglichkeit, gutes Schaumverhalten und angenehme Haarpflege und -frisieren auszeichnen.

Es hat sich überraschend gezeigt, und darin liegt die Lösung dieser Aufgaben, dass kosmetische und dermatologische Zubereitung enthaltend eine Kombination von anionischen und nichtionischen Tensiden bestehend aus
a. Alkylpolyglucosiden (APG) der Formel

   R-O-(CH₂-CH₂O)ₙ-Zₓ
b. Alkyl(ether)sulfaten (AES) der Formel

   R-O-(CH₂- CH₂O)ₙSO₃M
c. PEG-Sorbitanester (PSE) der Formel 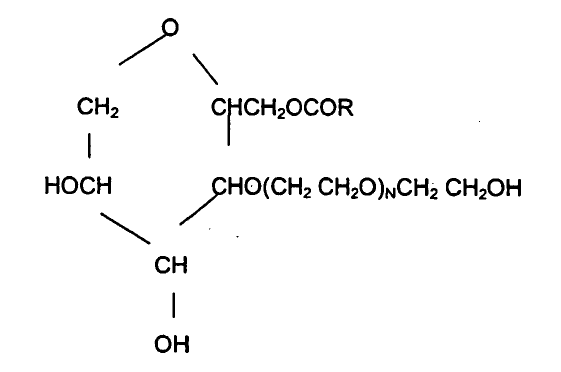 und
d. Alkylcitratsulfosuccinaten (ACS) der Formel 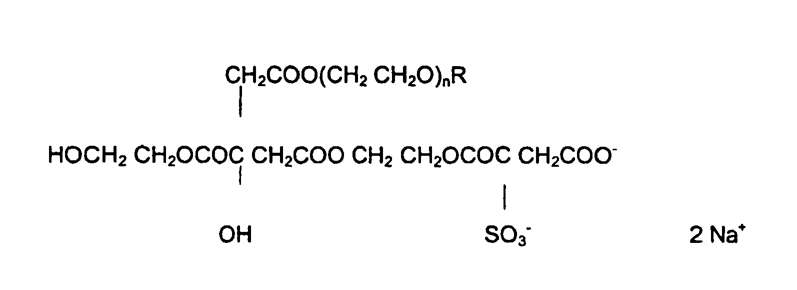 mit
   R = geradlinig oder verzweigte, gesättigte oder ungesättigte C₆-C₂₂ Kohlenwasserstoffkette; Z = Mono- oder Oligosaccharid; n = ganze Zahl von 0 - 10;
   x = ganze Zahl von 1 - 10; M = Alkali- oder Erdalkalimetall oder Ammonium;
   N = ganze Zahl von 0 - 60
   diese Aufgaben lösen.

Es war für den Fachmann nicht vorauszusehen gewesen, dass die erfindungsgemäßen Zubereitungen ein ausgezeichnetes Schaumverhalten, gute Reinigungsleistungen und vor allem gute Haut- und Haarverträglichkeit aufweisen. Insbesondere als Shampoo zeigten sich überraschend positive Eigenschaften, so wirkt das mit den erfindungsgemäßen Zubereitungen behandelte Haar äußerst gepflegt und lässt sich gut frisieren. Dies ist beispielsweise an der Kämmbarkeit, des Anscheins und der Fülle des Haares erkennbar.

Die erfindungsgemäße Kombination der anionischen und nichtionischen Tenside zeigt einen Synergismus, der für die überraschenderweise äußerst positiven Eigenschaften der kosmetischen Zubereitung verantwortlich ist.

In den aufgeführten Tensiden wird R in der Strukturformel vorteilhaft gewählt aus der Gruppe der unverzweigten Alkylreste, wobei der Hexyl-, der Heptyl-, der Octyl- der Nonyl- der Decyl-, der Undecyl-, der Dodecyl-, und der Tetradecylrest bevorzugt werden.

Die Gesamtmenge der anionischen und nichtionischen Tenside beträgt 8 - 15 Gew.%, insbesondere 10 - 13 Gew.%, bezogen auf die Gesamtzubereitung.

Das Tensidverhältnis der anionischenTenside (AES, ACS) zu den nichtionischen Tensiden (APG, PSE) beträgt vorteilhafterweise 20 : 80 bis 60 : 40, insbesondere 40 : 60 bis 55 : 45.

Weiterhin kann das Verhältnis von AES zu ACS 20 : 80 bis 60 : 40, insbesondere 40 : 60 bis 55 : 45 und/oder das Verhältnis von APG zu PSE 90 : 10 bis 50 : 50, insbesondere 80 : 20 bis 60 : 40 gewählt werden. Durch diese innovative Tensidkombination und Mengenverhältniswahl resultieren die angesprochenen positiven Eigenschaften der erfindungsgemäßen Zubereitungen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist in kosmetischen und/oder dermatologischen Zubereitungen im allgemeinen bevorzugt. Erfindungsgemäß können als günstige Antioxidantien alle für kosmetische und/oder dermatologische Anwendungen geeigneten oder gebräuchlichen Antioxidantien verwendet werden.
Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, ψ-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), femer (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft deren jeweilige Konzentrationen aus dem Bereich von 0,001 - 10 Gew.-%, bezogen auf das Gesamtgewicht der Formulierung, zu wählen.

Die Herstellung der erfindungsgemäßen kosmetischen und/oder dermatologischen Zubereitungen erfolgt in der dem Fachmann bekannten üblichen Weise, zumeist dergestalt, dass die erfindungsgemäß verwendeten Substanzen oder Vorlösung dieser Substanzen bei gleichmäßigem Rühren und ggf. unter Erwärmen gelöst bzw. dispergiert werden.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele 1 - 3

| Conditioner-Shampoo mit Perlglanz | | | |
|---|---|---|---|
| | **1** | **2** | **3** |
| Polymer JR 400¹ | 0,5 | 0,5 | 0,5 |
| Texapon K 14 S spezial² | 4,0 | 5,0 | 4,5 |
| Plantacare 2000³ | 9,0 | 8,0 | 8,5 |
| Perlglanzmittel | 2,0 | 2,0 | 2,0 |
| Rewopol SBCS 50⁴ | 6,0 | 5,5 | 6,2 |
| Atlas G-4280⁵ | 3,2 | 3,5 | 3,0 |
| Konservierungsmittel, Parfüm, Verdicker, pH-Einstellung und Lösungsvermittler | q.s. | q.s. | q.s. |
| Wasser, VES (vollentsalzt) | ad 100,0 | ad 100,0 | ad 100,0 |

Der pH-Vert wird auf 6 eingestellt.

### Beispiele 4 - 6

| klares Conditioner-Shampoo | | | |
|---|---|---|---|
| | **4** | **5** | **6** |
| Polymer JR 400¹ | 0,5 | 0,5 | 0,5 |
| Texapon K 14 S spezial² | 4,2 | 5,0 | 4,8 |
| Plantacare 2000³ | 8,8 | 8,0 | 8,3 |
| Rewopol SBCS 50⁴ | 6,2 | 5,7 | 6,3 |
| Atlas G-4280⁵ | 3,0 | 3,5 | 3,1 |
| Komplexmittel, Konservierungsmittel, Parfüm, Verdicker, pH-Einstellung und Lösungsvermittler | q.s. | q.s. | q.s. |
| Wasser, VES | ad 100,0 | ad 100,0 | ad 100,0 |

Der pH-Wert wird auf 6 eingestellt.

### Beispiele 7 - 9

| klares Light-Shampoo mit Volumeneffekt | | | |
|---|---|---|---|
| | **7** | **8** | **8** |
| Texapon K 14 S spezial² | 4,0 | 5,0 | 4,5 |
| Plantacare 2000³ | 9,2 | 8,0 | 8,4 |
| Rewopol SBCS 50⁴ | 6,0 | 5,5 | 6,2 |
| Atlas G-4280⁵ | 3,0 | 3,5 | 3,2 |
| Komplexbildner,Konservierungsmittel, Parfüm, Verdicker, pH-Einstellung und Lösungsvermittler | q.s. | q.s. | q.s. |
| Wasser, VES | ad 100,0 | ad 100,0 | ad 100,0 |

| | | | |
|---|---|---|---|
| Der pH-Wert wird auf 5,5 eingestellt. ¹ kationisches Cellulosederivat der Fa. Amerchol, ² Myrethsulfat 70%ig der Fa. Cognis (AES) ³ Decylgucoside 50%ig der Fa. Cognis (APG) ⁴ Citronensäurealkylpolyglykolester-sulfosuccinat, Dinatriumsalz 38%ig der Fa. Goldschmidt (ACS) ⁵ PEG-80 Sorbitan Laurate 73%ig der Fa. Uniqema (PSE) | | | |

## Patentansprüche

1. Kosmetische Zubereitung enthaltend eine Kombination von anionischen und nichtionischen Tensiden bestehend aus
a. Alkylpolyglucosiden (APG) der Formel
R-O-(CH₂- CH₂O)ₙ-Zₓ
b. Alkyl(ether)sulfaten (AES) der Formel
R-O-(CH₂- CH₂O)ₙSO₃M
c. PEG-Sorbitanester (PSE) der Formel 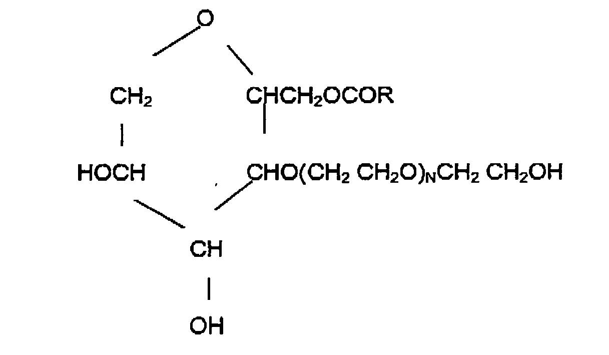 und
d. Alkylcitratsulfosuccinaten (ACS) der Formel 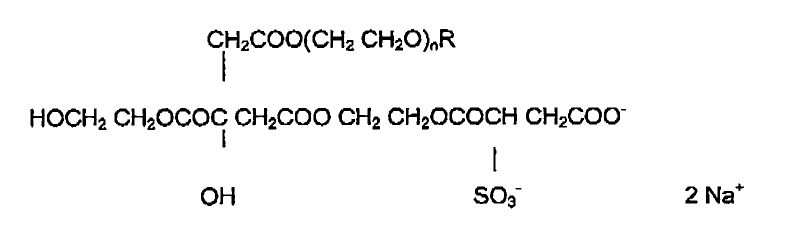 mit R = geradlinig oder verzweigte, gesättigte oder ungesättigte C₆-C₂₂ Kohlenwasserstoffkette; Z = Mono- oder Oligosaccharid; n = ganze Zahl von 0 - 10;
x = ganze Zahl von 1 - 10; M = Alkali- oder Erdalkalimetall oder Ammonium;
N = ganze Zahl von 0 - 60.
mit
R = geradlinig oder verzweigte, gesättigte oder ungesättigte C₆-C₂₂ Kohlenwasserstoffkette; Z = Mono- oder Oligosaccharid; n = ganze Zahl von 0 - 10;
x = ganze Zahl von 1 - 10; M = Alkali- oder Erdalkalimetall oder Ammonium;
N = ganze Zahl von 0 - 60

2. Zubereitungen nach Anspruch 1, **dadurch gekennzeichnet, dass** R in der Strukturformel gewählt wird aus der Gruppe der unverzweigten Alkylreste, insbesondere aus den Hexyl- , Heptyl-, Octyl-, Nonyl-, Decyl-, Undecyl-, Dodecyl- und/oder den Tetradecylresten.

3. Zubereitungen nach Anspruch 1 oder 2, **dadurch gekennzeichnet, dass** die Gesamtmenge der Tenside 8 - 15 Gew.%, insbesondere 10 - 13 Gew.%, bezogen auf die Gesamtzubereitung beträgt

4. Zubereitungen nach Anspruch 1, 2 oder 3, **dadurch gekennzeichnet, dass** das Tensidverhältnis anionischesTensid (AES, ACS) zu nichtionisches Tensid (APG, PSE) 20 : 80 bis 60 : 40, insbesondere 40 : 60 bis 55 : 45 beträgt.

5. Zubereitungen nach einem der vorstehenden Ansprüche, **dadurch gekennzeichnet, dass** das Verhältnis von AES : ACS 20 : 80 bis 60 : 40, insbesondere 40 : 60 bis 55 : 45 und/oder das Verhältnis von APG : PSE 90 : 10 bis 50 : 50, insbesondere 80 : 20 bis 60 : 40 beträgt.

6. Haarshampoo enthaltend Zubereitungen nach einem Ansprüche 1 bis 5.

7. Verwendung der Zubereitungen nach den Ansprüchen 1 bis 5 als Reinigungsmittel für Haut und/oder Haare, insbesondere als Haarshampoo.

## Claims

1. Cosmetic preparation comprising a combination of anionic and nonionic surfactants consisting of
a. alkyl polyglucosides (APGs) of the formula
R-O-(CH₂-CH₂O)ₙ-Zₓ
b. alkyl (ether) sulphates (AESs) of the formula
R-O-(CH₂-CH₂O)ₙSO₃M
c. PEG sorbitan esters (PSEs) of the formula 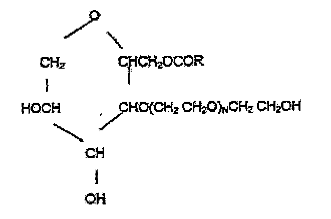 and
d. alkyl citrate sulphosuccinates (ACSs) of the formula 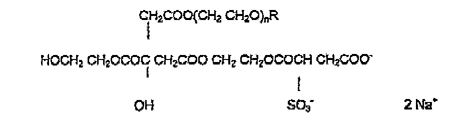
where R = straight-chain or branched, saturated or unsaturated C₆-C₂₂ hydrocarbon chain; Z = monosaccharide or oligosaccharide; n = integer from 0-10; x = integer from 1-10; M = alkali metal or alkaline earth metal or ammonium; N = integer from 0-60.

2. Preparations according to Claim 1, **characterized in that** R in the structural formula is chosen from the group of unbranched alkyl radicals, in particular from the hexyl, heptyl, octyl, nonyl, decyl, undecyl, dodecyl and/or the tetradecyl radicals.

3. Preparations according to Claim 1 or 2, **characterized in that** the total amount of the surfactants is 8-15% by weight, in particular 10-13% by weight, based on the total preparation.

4. Preparations according to Claim 1, 2 or 3, **characterized in that** the surfactant ratio of anionic surfactant (AES, ACS) to nonionic surfactant (APG, PSE) is 20:80 to 60:40, in particular 40:60 to 55:45.

5. Preparations according to one of the preceding claims, **characterized in that** the ratio of AES:ACS is 20:80 to 60:40, in particular 40:60 to 55:45, and/or the ratio of APG: PSE is 90:10 to 50:50, in particular 80:20 to 60:40.

6. Hair shampoo comprising preparations according to one of Claims 1 to 5.

7. Use of the preparations according to Claims 1 to 5 as cleaning agent for skin and/or hair, in particular as hair shampoo.

## Revendications

1. Préparation cosmétique contenant une combinaison d'agents tensioactifs anioniques et non ioniques constitués par
a. des alkylpolyglucosides (APG) de formule
R-O-(CH₂-CH₂O)ₙ-Zₓ
b. des alkyl(éther)sulfates (AES) de formule
R-O-(CH₂-CH₂O)ₙSO₃M
c. des esters de PEG-sorbitane (PSE) de formule 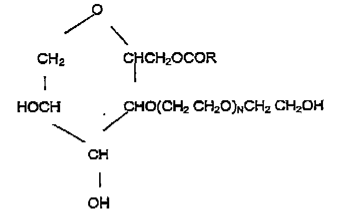 et
d. des alkylcitratesulfosuccinates (ACS) de formule 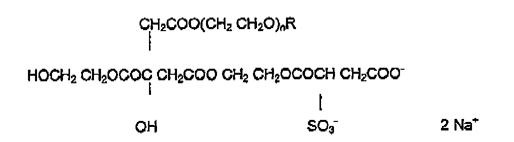 avec R = une chaîne hydrocarbonée linéaire ou ramifiée, saturée ou insaturée en C₆-C₂₂ ; Z = un monosaccharide ou un oligosaccharide ; n = un nombre entier de 0-10 ; x = un nombre entier de 1-10 ; M = un métal alcalin ou alcalino-terreux ou ammonium ; N = un nombre entier de 0-60.

2. Préparations selon la revendication 1, **caractérisées en ce que** R dans la formule de structure est choisi dans le groupe des radicaux alkyle non ramifiés, en particulier parmi les radicaux hexyle, heptyle, octyle, nonyle, décyle, undécyle, dodécyle et/ou tétradécyle.

3. Préparations selon la revendication 1 ou 2, **caractérisées en ce que** la quantité totale des agents tensioactifs est de 8-15% en poids, en particulier de 10-13% en poids, par rapport à la préparation totale.

4. Préparations selon la revendication 1, 2 ou 3, **caractérisées en ce que** le rapport tensioactif d'agent tensioactif anionique (AES, ACS) à agent tensioactif non ionique (APG, PSE) est de 20 : 80 à 60 : 40, en particulier de 40 : 60 à 55 : 45.

5. Préparations selon l'une quelconque des revendications précédentes, **caractérisées en ce que** le rapport de AES : ACS est de 20 : 80 à 60 : 40, en particulier de 40 : 60 à 55 : 45 et/ou le rapport de APG : PSE est de 90 : 10 à 50 : 50, en particulier de 80 : 20 à 60 : 40.

6. Shampooing pour cheveux contenant des préparations selon l'une quelconque des revendications 1 à 5.

7. Utilisation des compositions selon les revendications 1 à 5 comme agent de nettoyage pour la peau et/ou les cheveux, en particulier comme shampooing pour cheveux.
